(19) Europäisches Patentamt
European Patent Office
Office européen des brevets

(11) Publication number: **0 426 574 A1**

(12) **EUROPEAN PATENT APPLICATION**

(21) Application number: **90403111.9**

(22) Date of filing: **02.11.90**

(51) Int. Cl.[5]: **C07D 513/04**, A61K 31/44, //(C07D513/04,277:00,221:00)

(30) Priority: **02.11.89 JP 284911/89**

(43) Date of publication of application: **08.05.91 Bulletin 91/19**

(84) Designated Contracting States: **BE CH DE FR GB IT LI NL SE**

(71) Applicant: **TERUMO KABUSHIKI KAISHA**
**No. 44-1, Hatagaya 2-chome, Shibuya-ku**
**Tokyo 151(JP)**

(72) Inventor: **Shikata, Yoshiyuki**
**c/o Terumo K.K., 1500 Inokuchi, Nakai-machi**
**Ashigarakami-gun, Kanagawa-ken(JP)**
Inventor: **Endo, Isamu**
**c/o Terumo K.K., 1500 Inokuchi, Nakai-machi**
**Ashigarakami-gun, Kanagawa-ken(JP)**
Inventor: **Koyama, Shingo**
**c/o Terumo K.K., 1500 Inokuchi, Nakai-machi**
**Ashigarakami-gun, Kanagawa-ken(JP)**

(74) Representative: **Gillard, Marie-Louise et al**
**Cabinet Beau de Loménie 55, Rue d'Amsterdam**
**F-75008 Paris(FR)**

(54) **Thiazole derivatives and antiulcer agents containing the same.**

(57) Novel thiazole derivatives represented by the formula (I) are disclosed. Representative examples of them include 2-methylamino[5,4-b]pyridothiazole, 2-phenylamino[5,4-b]pyridothiazole, 2-phenylamino[5,4-b]-pyridothiazole and 2-benzylamino[5,4-b]pyridothiazole. The thiazole derivatives possess an antiulcer activity.

The thiazole derivatives can be prepared by reacting isothiocyanate with 3-amino-2-chloropyridine or reacting an amine derivative with 2-chloro-3-isothiocyanatopyridine.

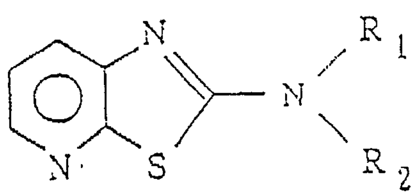

(I)

EP 0 426 574 A1

# THIAZOLE DERIVATIVES AND ANTIULCER AGENTS CONTAINING THE SAME

## Background of the Invention

### 1. Field of the Invention

This invention relates to novel thiazole derivatives and their pharmaceutically acceptable salts.

The invention also relates to pharmaceutical compositions comprising the thiazole derivatives or their pharmaceutically acceptable salts.

### 2. Description of the Prior Art

There have heretofore been known various antiulcer agents, but their efficacies are not fully satisfactory, and development of antiulcer agents with superior efficacies has been desired.

As a result of syntheses of various thiazole derivatives and extensive studies on their physiological actions, we have found that the thiazole derivatives of the invention inhibit the formation of an ethanol hydrochloric acid ulcer which is an ulcer model in animals.

It is therefore an object of the invention to provide novel thiazole derivatives and pharmaceutically acceptable salts thereof.

A further object of the invention is to provide antiulcer agents containing the above derivatives or their pharmaceutically acceptable salts.

## Detailed Description of the Invention

This invention is directed to a thiazole derivative represented by the formula (I)

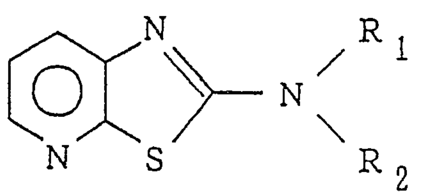

( I )

wherein $R_1$ and $R_2$ which may be the same or different represent a hydrogen atom; a lower alkyl group optionally having, as a substituent, phenyl, thienyl, pyridyl or naphthyl; or a phenyl group optionally having from one to three substituents selected from lower alkoxycarbonyl, hydroxy, lower alkoxy, amino, mono- or di(lower)alkylamino and halogen,except that both $R_1$ and $R_2$ are hydrogen atoms. Further, the invention is directed to antiulcer agents comprising the thiazole derivative of the formula (I) or pharmaceutically acceptable salts thereof.

Examples of the salts include a salt with an inorganic acid such as the hydrochloride and the sulfate and a salt with an organic acid such as the acetate and the tartrate.

In the above definition of $R_1$ and $R_2$ the lower alkyl group means an alkyl group having 1-4 carbon atoms such as methyl, ethyl, n-propyl, isopropyl, n-butyl, isobutyl or tert-butyl. These alkyl groups may be substituted with phenyl, thienyl, pyridyl or naphthyl. The term "lower alkoxy" means an alkoxy group with the alkyl moiety being a lower alkyl group as above defined.

The above-mentioned thiazole derivatives (I) are produced by

(a) reacting an isothiocyanate derivative represented by the formula (II)

R-NCS (II)

wherein R represents a lower alkyl group optionally having as a substituent phenyl, thienyl, pyridyl or naphthyl; or a phenyl group optionally having from one to three substituents selected from lower alkoxycarbonyl, hydroxy, lower alkoxy, amino, mono- or di(lower)alkylamino and halogen with 3-amino-2-chloropyridine, or

(b) reacting an amine derivative having the formula (III)

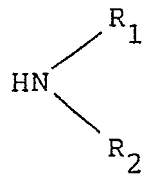

(III)

wherein $R_1$ and $R_2$ have the same meanings as set forth above with 2-chloro-3-isothiocyanatopyridine.

The above reactions are carried out by a conventional method. For example, the reactions are conveniently conducted by dissolving the compound (II) or (III) in an appropriate organic solvent such as methanol, ethanol, dichloromethane, carbon tetrachloride, benzene, toluene, ether or tetrahydrofuran in an atmosphere of an innert gas such as nitrogen or argon, adding to the solution 3-amino-2-chloropyridine or 2-chloro-3-isothiocyanatopyridine and allowing the mixture to react at from room temperature to reflux temperature of a solvent used for 30 min. to 24 hours.

After the completion of the reaction the precipitates are collected on a filter, and if needed, purified by recrystallization or column chromatography.

It is noted that, according to the process (a) as described above, a compound of the formula (I) wherein either one of $R_1$ and $R_2$ represents a hydrogen atom is prepared.

The thiazole derivatives (I) of the invention are used as an antiulcer agent. The dosage is generally 10-2000 mg, preferably 20-600 mg per day for a male adult though variable depending upon conditions of the patient. Preferably, it is divided into one to three doses per day as needed by conditions of the patient. Route of administration may be in any form suitable for the administration. Oral administration is especially desirable, intravenous administration being also feasible.

The compounds of the invention can be administered either alone or in admixture, as one of the active ingredients, with pharmaceutical carriers or excipients and other additives by a conventional method, in various forms such as tablets, sugar-coated tablets, powders, capsules, granules, suspension, emulsion and injectables. Examples of the carrier or excipient are calcium carbonate, calcium phosphate, starch, glucose, lactose, dextrin, alginic acid, mannitol, talc and magnesium stearate.

Examples and test examples are given below to describe the invention in more detail, but the invention is to be in no way limited thereto.

Example 1

In an atmosphere of argon 5.00 g of 3-amino-2-chloropyridine and 2.80 g of methylisothiocyanate were dissolved in 25 ml of ethanol, and the solution was heated under reflux for 10 hours. Crystals precipitated were separated by filtration and washed with ethanol. There was produced 3.83 g of 2-methylamino[5,4-b]-pyridothiazole hydrochloride. Spectroscopic data of the product support structure of the formula (IV) below.

NMR (DMSO d-6) δ:

3.06(3H,s), 7.1-8.3(3H,m)

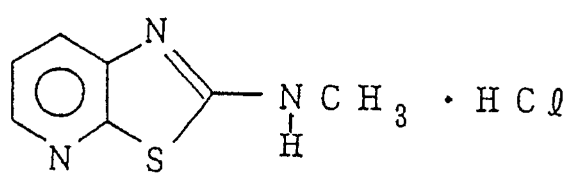

(IV)

Example 2

In an atmosphere of argon 2.00 g of 3-amino-2-chloropyridine and 2.10 g of phenylisothiocyanate were dissolved in 20 ml of ethanol, and the solution was heated under reflux for 16 hours. Crystals precipitated were separated by filtration and washed with ethanol. There was produced 2.13 g of 2-phenylamino[5,4-b]-pyridothiazole hydrochloride. Spectroscopic data of the product support structure of the formula (V) below.

NMR (DMSO d-6) δ:
6.9-7.5(4H,m), 7.7-8.3(4H,m)

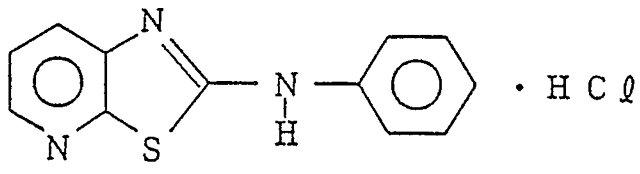

(V)

Example 3

In an atmosphere of argon 2.00 g of 3-amino-2-chloropyridine and 2.33 g of benzylisocyanate were dissolved in 20 ml of ethanol, and the solution was heated under reflux for 8 hours. Crystals precipitated were separated by filtration and washed with ethanol. There was produced 2.46 g of 2-benzylamino[5,4-b]-pyridothiazole hydrochloride. Spectroscopic data of the product support structure of the formula (VI) below.
NMR (DMSO d-6) δ:
4.82(2H,bs), 6.9-8.2(8H,m)

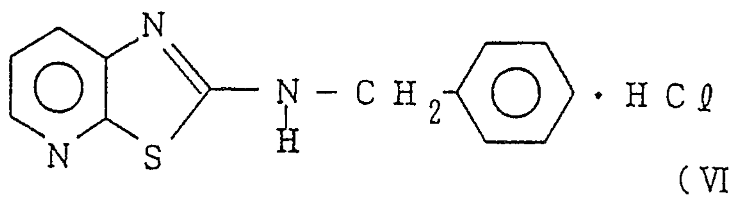

(VI)

Example 4

In an atmosphere of argon 1.50 g of 3-amino-2-chloropyridine and 2.51 g of 4-dimethylaminophenylisothiocyanate were dissolved in 25 ml of ethanol, and the solution was heated under reflux for 16 hours. Crystals precipitated were separated by filtration and washed with ethanol. There was produced 0.48 g of 2-(4-dimethylaminophenyl)amino[5,4-b]pyridothiazole hydrochloride. Spectroscopic data support structure of the formula (VII) below.
NMR (DMSO d-6) δ:
3.18(6H,bs), 7.2-8.3(7H,m)

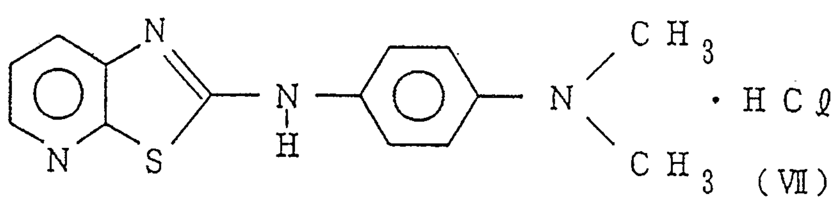

(VII)

Example 5

In an atmosphere of argon 2.00 g of 3-amino-2-chloropyridine and 2.54 g of 4-methoxyphenylisothiocyanate were dissolved in 40 ml of ethanol, and the solution was heated under reflux for 16 hours. Crystals precipitated were separated by filtration and washed with ethanol. There was produced 1.65 g of 2-(4-methoxyphenyl)-amino[5,4-b]pyridothiazole hydrochloride. Spectroscopic data of the product support structure of the formula (VIII) below.

NMR (DMSO d-6) δ:
3.90(3H,s), 7.2-8.3(8H,m)

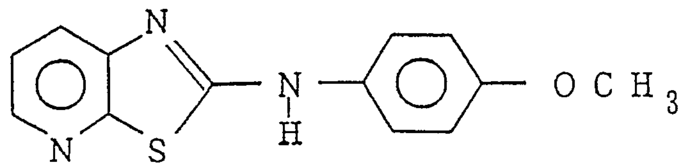
(VIII)

[Test example]

Inhibitory activity against ethanol hydrochlorio acid ulcer

To SD male rats (weighing 150-250 g) fasted for 24 hours was administered orally a thiazole derivative of the invention. One hour later, a hydrochloric acid solution in ethanol (containing 150 mM hydrochloric acid in 60% ethanol) was orally given in a volume of 0.5 ml/100 g bodyweight.

One hour later, the animals were sacrificed with ether. The stomach was excised and, after formalin treatment, measured for the length (mm) of lesions developed in glandular portions of the stomach. Total length of the lesions per animal was taken as ulcer index.

As shown in the table, marked antiulcer activities were found in the test. It was demonstrated that isoprenoid derivatives of the invention not shown in the table also have a similar antiulcer activity.

Percent inhibition of ulceration as referred to in the table is a value obtained by reducing from 1 the ulcer index for the rat with a thiazole derivative of the invention orally given divided by the ulcer index for the orally non-administered rat and multiplying the value by 100. The calculation is conducted by the formula shown below.

$$\text{Percent inhibition of ulceration (\%)} = \left(1 - \frac{\text{Ulcer index for thiazole derivative orally administered rat}}{\text{Ulcer index for thiazole derivative orally non-administered rat}}\right) \times 100$$

Table Inhibitory activity against ethanol hydrochloric acid ulcer

| Structural formula | Example No. | Dose | Percent inhibition of ulceration (%) |
|---|---|---|---|
| 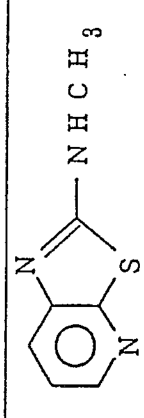 | 1 | 30mg/kg | 100.0 |
| 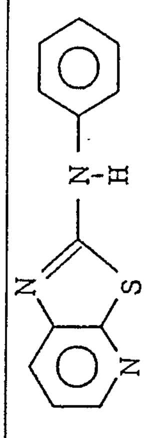 | 2 | 〃 | 88.8 |
| 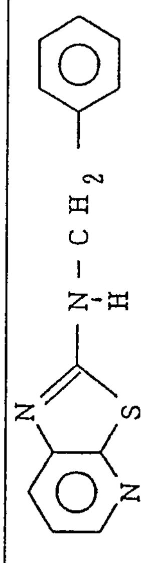 | 3 | 〃 | 87.2 |
| 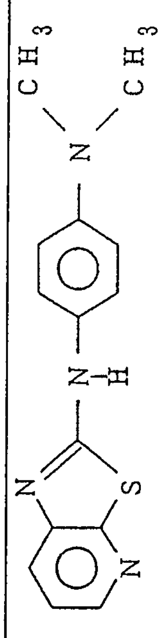 | 4 | 〃 | 45.5 |
| 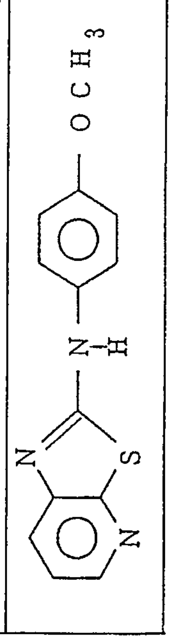 | 5 | 〃 | 51.2 |

(Acute toxicity)

An acute toxicity text by oral administration was run using ICR male mice (5 week-old). The $LD_{50}$ value was 2500 mg/kg or higher with any of the compounds of the invention thereby confirming higher safety than the effective dose.

**Claims**

1) A thiazole derivative represented by the formula (I)

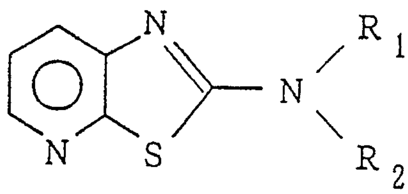

( I )

wherein $R_1$ and $R_2$ which may be the same or different represent a hydrogen atom; a lower alkyl group optionally having as a substituent phenyl, thienyl, pyridyl or naphthyl; or a phenyl group optionally having from one to three substituents selected from lower alkoxycarbonyl, hydroxy, lower alkoxy, amino, mono- or di(lower)alkoxyamino and halogen except that both $R_1$ and $R_2$ are hydrogen atoms, or a pharmaceutically acceptable salt thereof.

2) A thiazole derivative according to Claim 1 wherein one of $R_1$ and $R_2$ is a hydrogen atom and the other is a lower alkyl group, phenyl, benzyl, di(lower)alkylaminophenyl or lower alkoxyphenyl.

3) An antiulcer agent comprising a medicinally effective amount of the thiazole derivative according to Claim 1.

4. An antiulcer pharmaceutical composition comprising a therapeutically effective amount of the thiazole derivative according to Claim 1 and a pharmaceutical carrier therefor.

European Patent Office

# EUROPEAN SEARCH REPORT

Application Number

**EP 90 40 3111**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | JOURNAL OF HETEROCYCLIC CHEMISTRY, vol. 14, no. 1, February 1977, pages 129-134, Hetero Corp., Utah, US; H.W. ATLAND et al.: "A facile synthesis of 2-aminothiazolo[5,4-b]- and 2-aminothiazolo[4,5-c] pyridines" * Page 130, table I; page 131, table II * | 1 | C 07 D 513/04<br>A 61 K 31/44 //<br>(C 07 D 513/04<br>C 07 D 277:00<br>C 07 D 221:00 ) |
| X | CHEMICAL ABSTRACTS, vol. 85, no. 21, 22nd November 1976, page 538, abstract no. 159963t, Columbus, Ohio, US; B.G. KHADSE et al.: "Synthesis and study of 2-arylaminopyrido(3,2-d)thiazoles as possible anthelmintic agents", & BULL. HAFFKINE INST. 1976, 4(1), 16-19 * Abstract * | 1 | |
| X | CHEMICAL ABSTRACTS, vol. 96, no. 15, 12th April 1982, page 690, abstract no. 122683c, Columbus, Ohio, US; P.P. CHAUDHARI et al.: "Synthesis and anthelmintic activity of new pyrido[3,2-d]thiazoles", & BULL. HAFFKINE INST. 1980, 8(3), 87-92 * Abstract * | 1 | |
| A | FR-A-2 556 725 (FARMITALIA CARLO ERBA) * Claim 1; page 8, lines 17-28 * | 1,3 | |

TECHNICAL FIELDS SEARCHED (Int. Cl.5)

C 07 D 513/00
A 61 K 31/00

The present search report has been drawn up for all claims

| Place of search | Date of completion of search | Examiner |
|---|---|---|
| The Hague | 01 February 91 | VOYIAZOGLOU D. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention
E: earlier patent document, but published on, or after the filing date
D: document cited in the application
L: document cited for other reasons
&: member of the same patent family, corresponding document